# EUROPEAN PATENT APPLICATION

(11) **EP 4 431 164 A1**
(43) Date of publication of application: **18.09.2024**
(21) Application number: 24162766.0
(22) Date of filing: 11.03.2024
(51) Int. Cl.: A63B 24/00, A63B 21/005, G06Q 10/00, G06Q 10/06, G06Q 50/10, G06Q 30/0208, G06Q 40/04, A63B 71/06

(54) **SYSTEM FOR THE INTERACTIVE ACTIVATION OF AN ELECTRONIC DEVICE**

(30) Priority: 13.03.2023 IT 202300004674
(71) Applicant: Progetto Design & Build S.r.l., 20143 Milano (MI) (IT)
(72) Inventor: Notarbartolo, Massimiliano, 20143 Milano MI (IT)
(74) Representative: Perani & Partners S.p.A.

(57) **Abstract**

A system for the interactive activation of an electronic device (2), comprising at least one electronic device (2) connected to a power supply or radio frequency; at least one interactive apparatus (3) usable by a user (U) to perform a motor activity according to a motor program, the interactive apparatus (3) being adapted to receive data relating to the motor program; and a control unit (4) placed in signal communication with the interactive apparatus (3) and configured to receive data relating to the motor program from the interactive apparatus (3), wherein the motor program comprises parameters indicating motor activity relating to the interactive apparatus (3), the interactive apparatus (3) is configured to generate an electrical command signal upon reaching a threshold relating to the parameters indicating motor activity, the control unit (4) is configured to receive the electrical command signal and generate an electrical unlocking signal, and the electronic device (2) is configured to switch from a locking to an unlocking configuration upon receiving the electrical unlocking signal.

## Description

### Technical Field

The present invention relates to a system for the interactive activation of an electronic device. In particular, such a system may be applied, for example, in a home or office environment.

### Description of the prior art

The high diffusion of a sedentary lifestyle within the population, leading to high associated risks, including increased cases of hypertension, high blood cholesterol, overweight and diabetes, as well as diseases of the osteoskeletal system, muscular system, connective tissue and possible increases in stress, depression and generalised anxiety is well known. The risks associated with a sedentary lifestyle can be contrasted by practising light physical activity that can bring physical benefits to an individual's health.

The causes underlying the diffusion of sedentary behaviour are also related to an increase in jobs that require a high level of sedentary activity for long periods of time, resulting in a reduction of free time that can be devoted to exercise.

In order to partially contrast sedentary behaviours, the creation of environments dedicated to light, moderate or vigorous physical activity in home and working contexts is known in the background art. In particular, home or working environments are known to be provided with gym or physical exercise equipment. Such environments are typically associated with structured physical activity based on planned motor programs comprising specific goals.

Indeed, it is well known that interspersing daily activities or working activities with physical activity brings benefits to everyone's health. In addition, it allows everyone to better manage his or her free time.

### Problem of the prior art

However, in the prior art, the use of special training environments requires targeted, voluntary, and structured physical activity to be performed in relatively short periods of time. As a result, many individuals voluntarily choose not to use or create such spaces due to high physical exertion to be performed during working hours or during daily actions, in addition to a high amount of time required to perform physical activity in such spaces.

Therefore, the need is felt in the prior art to gradually introduce daily physical activity, in a way that is customised for everyone, without affecting the individual's everyday life. In particular, in working contexts, there is a need to perform physical activity without excessively affecting normal tasks and without requiring a long time.

### Summary of the invention

The object of the present invention is to implement a system capable of making physical activity possible without affecting the everyday life.

More specifically, the object of the present invention is to implement a system that enables a user to perform light, unstructured physical activity in a home or working context.

The specified technical task and the specified objects are substantially achieved by a system comprising the technical characteristics set forth in claim 1.

### Advantages of the invention

Thanks to an embodiment, it is possible to obtain a system that takes advantage of an interactive apparatus to perform physical activity according to a motor program, in order to activate and enable the use of an electronic device.

Advantageously, the system comprises a control unit capable of customising the motor program for each user, in order to allow each user to perform physical activity by means of the interactive apparatus on the basis of their respective state of health. In more detail, the system allows each user to perform unstructured motor activity, thus integrating the performance of light physical activity by means of the interactive apparatus with the daily activities of each user.

Still advantageously, the system may comprise a plurality of electronic devices associated with a plurality of interactive apparatuses, in order to perform, during working or daily routine tasks, multiple motor activities according to respective motor programs. In detail, the system can associate a tracking parameter with each motor activity that can be performed by means of the interactive apparatus to track the overall motor activity performed by the user over a period of time.

### BRIEF DESCRIPTION OF THE DRAWINGS

The characteristics and advantages of the present invention will become clearer from the following detailed description of a possible preferred embodiment, shown by way of a non-limiting example in the set of drawings, in which:
- Figure 1 schematically shows a system according to the present invention,
- Figure 2 schematically shows an embodiment of the system of Figure 1.

### DETAILED DESCRIPTION

The present invention relates to a system 1 for the interactive activation of an electronic device 2, comprising at least one electronic device 2 that can be powered by electrical energy. Preferably, the electronic device 2 comprises a household appliance.

In the context of the present invention, a household appliance is understood to be any appliance for domestic use in a home or office environment which is operated by means of a direct power supply or, alternatively, by means of electrical energy induced via radio frequency (e.g., by means of Bluetooth technology). By way of example, a Bluetooth-powered electronic device 2 is a sensorized padlock. Throughout this description, where not otherwise specified, "electronic device" will refer primarily to a device powered by direct power supply.

Still preferably, the electronic device 2 comprises one or more among air dispenser, liquid dispenser, soap dispenser, food and/or non-food dispenser, microwave and/or any device for heating or cooking food, sensorized padlock. Further preferably, the electronic device 2 may comprise a home automation system or a device capable of interacting with a home automation system.

The system 1 further comprises at least one interactive apparatus 3 usable by a user U to perform a motor activity according to a motor program. Preferably, the motor activity is of the cardio, muscular and/or joint type.

In the context of the present invention, a motor program refers to a series of motor actions and goals to be reached by the user U.

The interactive apparatus 3 is configured to receive data relating to the motor program.

According to a first embodiment of the invention, the interactive apparatus 3 is placed in signal communication with the electronic device 2. Preferably, the signal communication occurs via cable or wirelessly by means of wireless radio frequency network communication (e.g. Bluetooth and/or WiFi).

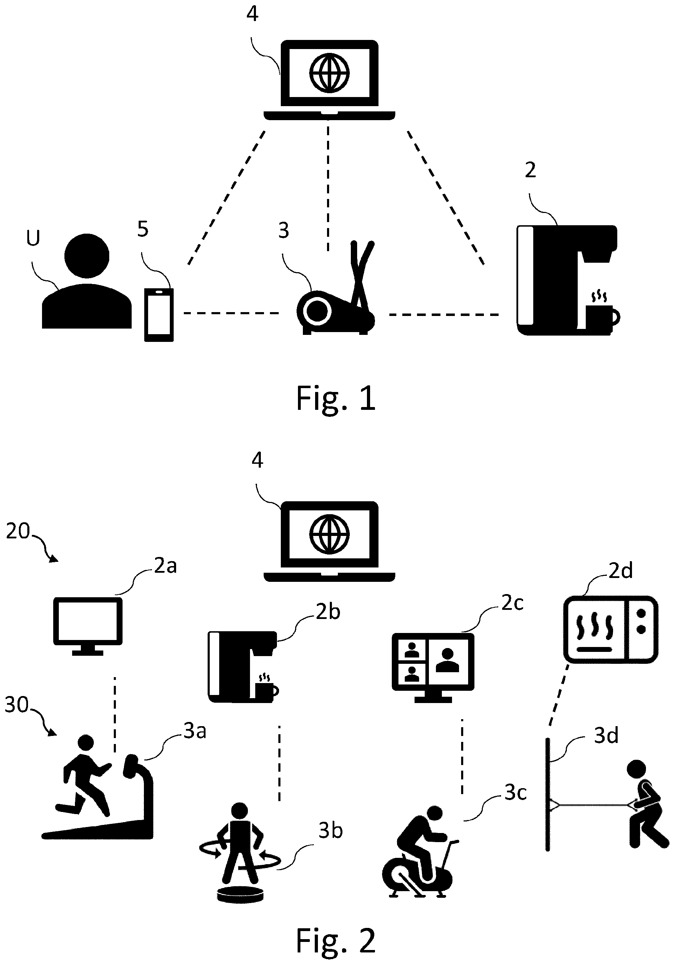

In accordance with the preferred embodiment of the invention, shown in Figures 1 and 2, the interactive apparatus 3 comprises a sports tool comprising one or more among stepper, pedalboard for legs and/or arms, device for rotating the torso, sensorized pull wall, sensorized pulley, treadmill, exercise bike.

The system 1 preferably comprises a control unit 4 placed in signal communication with the interactive apparatus 3 and configured to receive data relating to the motor program from the interactive apparatus 3. Preferably, the signal communication occurs via cable or wirelessly by means of wireless radio frequency network communication (e.g. Bluetooth and/or WiFi).

In accordance with the first embodiment of the invention, the control unit 4 is comprised inside the interactive apparatus 3 and is operable from the interactive apparatus 3.

In accordance with a second embodiment of the invention, the control unit 4 is a remote unit, external to the interactive apparatus 3, and is represented, by way of example, by a control unit 4 residing on a Cloud. Preferably, the control unit 4 is placed in signal communication with the electronic device 2.

In accordance with the second embodiment of the invention, the control unit 4 comprises a memory module, not shown, for storing data relating to the motor program. Furthermore, the control unit 4 is configured to process and update this data relating to the motor program.

In the second embodiment of the invention, the control unit 4 comprises a virtual cloud server for storing, archiving and processing data relating to the motor program. Note that the control unit 4 is also adapted to record data associated with each user U and to the interaction of each user U with the interactive apparatus 3, as detailed below.

Throughout the present description, where not otherwise specified, reference will be made to the second embodiment of the invention, wherein the control unit 4 is remote and external to the interactive apparatus 3.

The motor program comprises parameters indicating the motor activity associated with the interactive apparatus 3. Preferably, the parameters indicating motor activity comprise at least a number of repetitions of the motor activity and/or a time interval of performance of the motor activity. Still preferably, the parameters indicating motor activity further comprise the exertion intensity subjectively perceived. Note that the exertion intensity is defined in a range between light and moderate, according to values on the Borg scale.

Preferably, the time interval of performance of muscular and/or joint motor activity is between 20 and 60 seconds. More preferably, the time interval of performance of muscular and/or joint motor activity is between 20 and 45 seconds. Still preferably, the time interval of performance of cardio motor activity is 15 minutes or less.

The interactive apparatus 3 is configured to generate an electrical command signal upon reaching a threshold relating to the parameters indicating motor activity. Preferably, the threshold relating to the indicator parameters comprises at least a minimum value of the number of repetitions of the motor activity and/or a minimum value of the time interval of performance of the motor activity.

It should be noted that interactive apparatus 3 makes it possible to perform moderate and short movements that are compatible with other working or daily routine activities. In fact, the motor activity that can be performed by the user U enters in parallel into the workflow and/or daily routine without interfering with the working and/or daily routine tasks, as it is performed taking into account the exertion intensity perceived at an individual level by each user U.

The control unit 4 is configured to receive the electrical command signal and to generate an electrical unlocking signal.

The electronic device 2 is configured to switch from a locking configuration to an unlocking configuration upon receiving the electrical unlocking signal. In more detail, when not in use, the electronic device 2, even if connected to the power supply, is in a locking configuration and therefore is not usable or operable by a user U. The electronic device 2 is thus only usable in the unlocking configuration and, therefore, after performing the motor activity according to the motor program by means of the respective interactive apparatus 3 connected to the electronic device 2. Note that the electronic device 2 is only usable by the user U who has performed the motor activity by means of the interactive apparatus 3. As explained below in this description, in order to interact with the interactive apparatus 3 and, consequently, with the electronic device 2, the user U must first register and/or authenticate.

In accordance with the preferred embodiment of the invention, the interactive apparatus 3 is usable by a plurality of users. Preferably, each user U is associated with a respective motor program. Still preferably, the control unit 4 is configured to generate and process data relating to parameters indicating the motor activity for each user U. In particular, the control unit 4 is configured to generate the parameters indicating motor activity and the threshold of the motor program of each user U on the basis of predetermined clinical and physiological parameters of each user U.

It should be noted that the pre-established clinical and physiological parameters of each user U are collected and processed by means of suitable anthropometric measurements, such as BMI, height and/or weight of the U, validated tests and/or diagnostic assessments. In particular, the pre-established clinical and physiological parameters can be calculated by means of specific motor activities and exercises both in the user's healthy condition as well as in conditions of disease, disability, pregnancy and/or specific needs. Consequently, the generated motor program is specific to the needs, health status, lifestyle and motor skills of the respective user U. In particular, the generated motor program is generated taking into account whether the user U is sedentary, active or athletic. Note that the collection of the pre-established clinical and physiological parameters is carried out periodically to assess the health condition of the respective user U. Preferably, the collection of the pre-established clinical and physiological parameters of each user U is carried out every three months.

In accordance with the embodiment shown in Figure 2, the system 1 comprises a plurality of electronic devices 20. In addition, the system 1 preferably comprises a plurality of interactive apparatuses 30. In detail, each interactive apparatus 3a-3d is associated with a respective electronic device 2a-2d and is configured to activate the respective electronic device 2a-2d by means of the control unit 4. It should be noted that, by means of the control unit 4, it is also possible to change and interchange the connections between the plurality of electronic devices 20 and the plurality of interactive apparatuses 30, so as to associate, at different times, several interactive apparatuses 3 of the plurality of interactive apparatuses 30 to an electronic device 2.

According to an aspect of the invention, the motor program comprises a plurality of motor subprograms. Each motor subprogram is associated with a respective interactive apparatus 3a-3d and comprises parameters indicating motor activity related to the respective interactive apparatus 3a-3d.

According to the preferred embodiment of the invention, shown in Figure 1, the system 1 comprises a mobile device 5 placed in signal communication with the interactive apparatus 3 and/or the control unit 4.

Preferably, the signal communication between the mobile device 5 and the control unit 4 and/or between the mobile device 5 and the interactive apparatus 3 occurs via cable or wirelessly by means of wireless radio frequency network communication (e.g., Bluetooth and/or WiFi). Still preferably, the mobile device 5 is configured to receive data relating to the motor program from the control unit 4 and transmit data relating to the motor program, associated with the respective user U, to the control unit 4 and/or the interactive apparatus 3. In detail, the control unit 4 makes it possible to set up, process or modify the motor program associated with the respective user U on the basis of the interactive apparatus 3 used.

According to an embodiment of the invention, the mobile device 5 is adapted to generate a two-dimensional (e.g., barcode) or three-dimensional (e.g., QR code) code associated with the data relating to the motor program associated with each user U. Preferably, the interactive apparatus 3 comprises an optical reading device for detecting the two-dimensional or three-dimensional code generated by the mobile device 5. In more detail, the interactive apparatus 3 receives data relating to the motor program, and thus relating to the threshold to be reached, from the control unit 4 via the mobile device 5 of each user U.

Preferably, the mobile device 5 comprises a user interface, not shown, through which each user U can register and/or authenticate. In detail, the control unit 4 is adapted to receive from the mobile device 5 the data relating to the registration and/or authentication of the user U. Note that the data relating to the motor program associated with the registered and/or authenticated user are generated and calculated on the basis of the pre-established clinical and physiological parameters of the registered and/or authenticated user.

It should be noted that control unit 4 receives and stores data relating to the registered and/or authenticated user and the motor program associated with the registered and/or authenticated user in the memory module. In addition, the control unit 4 is configured to transmit and communicate this data in an explicit and protected form, via the mobile device 5, to the authenticated user. Please note that this data may be consulted in anonymous form by one or more administrators of the control unit 4 for statistical and scientific research purposes.

Preferably, the control unit 4 can modify the data relating to the respective motor program associated with the authenticated user on the basis of subjective feedback that can be entered by the authenticated user via the user interface. Such subjective feedback preferably comprises psycho-physical parameters relating to the exertion intensity subjectively perceived by the authenticated user and/or psycho-physical recovery with respect to the motor activity performed.

According to an aspect of the invention, the electronic device 3 is configured to switch from the unlocking configuration to the locking configuration after an operating time interval. Preferably, the operating time interval is between 30 and 90 seconds.

According to the preferred embodiment of the invention, the control unit 4 assigns, for each user U, a tracking parameter to the motor activity that can be performed by means of the interactive apparatus 3. Preferably, this tracking parameter is defined on the basis of the type of motor activity, i.e. cardio, muscular and/or joint activity. Still preferably, this tracking parameter can be modified by the control unit 4 on the basis of the subjective feedback entered by the authenticated user by means of the mobile device 5.

In accordance with the preferred embodiment of the invention, the control unit 4 associates the tracking parameter with a respective score related to the minutes of motor activity performed by the user U. In addition, the control unit 4 updates the score of the tracking parameter upon reaching the threshold. Note that the score associated with the tracking parameter makes it possible to track the overall motor activity performed over a period of time, e.g. a working day, by each user U. Preferably, each user U can preferably enter subjective feedback through the user interface of the mobile device 5 on the basis of the perceived exertion intensity upon reaching the threshold.

According to the preferred embodiment of the invention, the control unit 4 generates for each user U a target parameter to be achieved weekly or monthly by performing one or more motor activities according to a motor program. It should be noted that in order to have health benefits, an average, healthy adult person should perform a minimum of at least 150 minutes of moderate motor activity, or alternatively at least 75 minutes of vigorous motor activity. Preferably, the target parameter is calculated by considering one or more of the clinical and physiological parameters of each user U, the minimum time of motor activity required for each user U, the type of motor activity, the age of each user U, the gender of each user U, and values measured with a heart rate monitor for each user U. Still preferably, the control unit 4 associates the target parameter, by means of a conversion calculation, with a respective score relating to the minutes of motor activity to be performed weekly or monthly. The score relative to the target parameter is preferably between a minimum value of 130 minutes and a maximum value of 280 minutes.

According to the preferred embodiment of the invention, the control unit 4 stores the target parameter and the tracking parameter in the memory module. Still preferably, the control unit 4 compares the tracking parameter with the target parameter to track the weekly or monthly activity performed by each user U in order to assess the achievement of the score relative to the target parameter and, therefore, of the minimum time of physical activity relative to each user U.

According to an aspect of the invention, the control unit 4 can change the target parameter according to the subjective feedback entered by the user via the mobile device 5, so that the minimum time of physical activity relative to a respective user U can be adapted to the psycho-physical condition of said user U.

Below is an example of use of the system 1 according to the present invention.

According to the first embodiment of the invention, the use of the system 1 according to the present invention provides connecting the interactive apparatus 3 to the electronic device 2. More preferably, the use provides connecting each interactive apparatus 3a-3d to its respective electronic device 2a-2d.

According to the second embodiment of the invention, the use of the system 1 provides connecting the remote control unit 4 to the electronic device 2. Preferably, the use provides connecting the control unit 4 to each electronic device 2a-2d of the plurality of electronic devices 20.

Subsequently, the use of the system 1 provides connecting the mobile device 5 of the user U to the control unit 4 in order to receive in the mobile device 5 the data relating to the motor program associated with the user U. The control unit 4 then transmits data relating to the motor program associated with the user U to the interactive apparatus 3 by means of the respective mobile device 5.

Still preferably, the use of the system 1 thus provides performing a motor activity according to the motor program by means of the interactive apparatus 3. In detail, the use provides performing a motor activity according to a motor subprogram by means of the respective interactive apparatus 3a-3d of the plurality of interactive apparatuses 30. Thus, upon reaching the threshold relative to the parameters indicating the motor activity, the system 1 provides sending the electrical unlocking signal to the electronic device 2, in order to take the electronic device 2 into the unlocking configuration.

Still preferably, the use of the system 1 provides updating, by means of the control unit 4, the score relative to the tracking parameter associated with the relevant motor activity and comparing it with the score relative to the weekly or monthly target parameter. Finally, the use of the system 1 provides using the electronic device 2.

By way of example, a possible combination of an electronic device 2 of the plurality of electronic devices 20 with an interactive apparatus 3 of the plurality of interactive apparatuses 30 is hereinafter reported.

Preferably, the interactive apparatus 3 comprises a device for rotating the torso associated with an electronic device 2 comprising a coffee machine, or dispenser. The control unit 4 communicates the data relating to the motor program associated with the user U, continuously monitored and updated by the control unit 4, to the device for rotating the torso via the mobile device 5 used by the user U. In detail, the user U can authenticate in the mobile device 5 to consult data relating to the motor program. Thus, the user U can perform the motor activity by means of the device for rotating the torso and, upon reaching the threshold, the coffee machine is activated by the control unit 4 following the command signal generated by the device for rotating the torso. It should be noted, however, that according to an aspect of the invention, this combination can be modified by the control unit 4, which allows to associate a different interactive apparatus 3 of the plurality of interactive apparatuses 30 with the electronic device 2.

## Claims

1. System (1) for the interactive activation of an electronic device, comprising:
- at least one electronic device (2) that can be powered by electrical energy;
- at least one interactive apparatus (3) usable by a user (U) to perform a motor activity according to a motor program, the interactive apparatus (3) being adapted to receive data relating to the motor program; and
- a control unit (4) placed in signal communication with the interactive apparatus (3) and configured to receive data relating to the motor program from the interactive apparatus (3),
**characterized in that**
- the motor program comprises parameters indicating motor activity associated with the interactive apparatus,
- the interactive apparatus (3) is configured to generate an electrical command signal upon reaching a threshold relating to the parameters indicating motor activity,
- the control unit (4) is configured to receive the electrical command signal and to generate an electrical unlocking signal, and
- the electronic device (2) is configured to switch from a locking configuration to an unlocking configuration upon receiving the electrical unlocking signal.

2. System (1) according to claim 1, wherein:
- the parameters indicating motor activity comprise at least a number of repetitions of the motor activity and/or a time interval of performance of the motor activity,
- the threshold comprises at least a minimum value of the number of repetitions of the motor activity and/or a minimum value of the time interval of performance of the motor activity.

3. System (1) according to claim 1 or 2, wherein the interactive apparatus (3) is usable by a plurality of users, each user (U) being associated with a respective motor program, the control unit (4) being configured to generate and to process the data relating to the parameters indicating motor activity for each user (U).

4. System (1) according to claim 3, wherein the control unit (4) is configured to generate the parameters indicating motor activity and the threshold of the motor program of each user (U) on the basis of predetermined clinical and physiological parameters of each user (U).

5. System (1) according to any one of claims 1 to 4, wherein the electronic device (2) is configured to switch from the unlocking configuration to the locking configuration after an operating time interval.

6. System (1) according to any one of the preceding claims, comprising a plurality of electronic devices (20) and a plurality of interactive apparatuses (30), wherein:
- each interactive apparatus (3a, 3b, 3c, 3d) is associated with a respective electronic device (2a, 2b, 2c, 2d) and is configured to activate the respective electronic device (2a, 2b, 2c, 2d) by means of the control unit (4), and
- the motor program comprises a plurality of motor subprograms, each motor subprogram being associated with a respective interactive apparatus (3a, 3b, 3c, 3d) and comprising parameters indicating motor activity relating to the respective interactive apparatus (3a, 3b, 3c, 3d).

7. System (1) according to any one of the preceding claims, comprising a mobile device (5) placed in signal communication with the interactive apparatus (3) and/or with the control unit (4), the mobile device (5) being configured to receive the data relating to the motor program from the control unit (4) and to transmit data relating to the motor program to the control unit (4) and/or to the interactive apparatus (3).

8. System (1) according to claims 3 and 7, wherein:
- the mobile device (5) comprises a user interface through which each user (U) can register and/or authenticate, the control unit (4) being adapted to transmit to the mobile device (5) the data relating to the motor program associated with the registered and/or authenticated user.

9. System (1) according to any one of the preceding claims, wherein the electronic device (2) comprises one or more among air dispenser, liquid dispenser, soap dispenser, food and/or non-food dispenser, microwave and/or any device for heating or cooking food, sensorized padlock, home automation systems.

10. System (1) according to any one of the preceding claims, wherein the interactive apparatus (3) comprises a sports tool comprising one or more among exercise bike, stepper, pedalboard for legs and/or arms, device for rotating the torso, sensorized pull wall, sensorized pulley, treadmill.
